(19) Europäisches Patentamt
European Patent Office
Office européen des brevets

(11) Veröffentlichungsnummer: **0 141 119**
**A2**

(12) # EUROPÄISCHE PATENTANMELDUNG

(21) Anmeldenummer: **84110114.0**

(22) Anmeldetag: **24.08.84**

(51) Int. Cl.[4]: **C 07 D 249/14**
C 07 D 417/04, C 07 D 401/12
C 07 D 403/12, C 07 D 417/12
A 61 K 31/41, A 61 K 31/425

(30) Priorität: **06.10.83 DE 3336410**

(43) Veröffentlichungstag der Anmeldung:
**15.05.85 Patentblatt 85/20**

(84) Benannte Vertragsstaaten:
**AT BE CH DE FR GB IT LI LU NL SE**

(71) Anmelder: **LUDWIG HEUMANN & CO GMBH**
**Heideloffstrasse 18-28 Postfach 2260**
**D-8500 Nürnberg(DE)**

(72) Erfinder: **Schickander, Helmut, Dr. Dipl.-Chem**
**Moosäcker 25**
**D-8501 Eckental(DE)**

(72) Erfinder: **Herter, Rolf, Dr. Dipl.-Chem.**
**Bayernstrasse 42**
**D-8540 Schwabach(DE)**

(72) Erfinder: **Postius, Stefan, Dr.**
**Unschlittplatz 1**
**D-8500 Nürnberg(DE)**

(72) Erfinder: **Mörsdorf, Peter, Dr. Dipl.-Chem.**
**Untere Bahnhofstrasse 16**
**D-8501 Cadolzburg(DE)**

(72) Erfinder: **Szelenyi, Istvan, Dr.**
**Brahmsstrasse 16**
**D-8501 Schwaig(DE)**

(72) Erfinder: **Henning Ahrens, Kurt, Dr.**
**Praterstrasse 9**
**D-8500 Nürnberg(DE)**

(74) Vertreter: **Kraus, Walter, Dr. et al,**
**Patentanwälte Kraus, Weisert & Partner Irmgardstrasse 15**
**D-8000 München 71(DE)**

(54) **Sulfenamidderivate, Verfahren zu ihrer Herstellung und diese Verbindungen enthaltende Arzneimittel.**

(57) Es werden Sulfenamidderivate der allgemeinen Formel I beschrieben

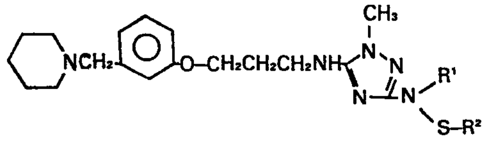

(I)

in der $R^1$ für ein Wasserstoffatom und $R^2$ für lineares oder verzweigtkettiges $C_{1-6}$-Alkyl oder $C_{5-6}$-Cycloalkyl oder für unsubstituiertes oder ein- bis dreifach substituiertes Aryl oder Heteroaryl steht oder wobei $R^1$ und $R^2$ zusammen einen Isothiazolinring oder Isothiazolinonring bilden, sowie die physiologisch annehmbaren Salze davon. Weiterhin werden ein Verfahren zur Herstellung dieser Verbindungen und sie enthaltende Arzneimittel beschrieben.

EP 0 141 119 A2

BESCHREIBUNG

Die Erfindung betrifft neue Sulfenamidderivate mit einer hohen selektiven Wirkung auf Histamin-$H_2$-Rezeptoren, Verfahren zu ihrer Herstellung und Arzneimittel, die diese Verbindungen enthalten, sowie schließlich die Verwendung dieser Verbindungen in der Therapie.

Als Antiulcusmittel haben Cimetidin und Ranitidin bereits Eingang in die Therapie gefunden. Die Halbwertszeiten von Cimetidin und Ranitidin sind jedoch verhältnismäßig kurz. Dadurch wird es nötig, mehrfach täglich Tabletten mit Dosiseinheiten von 160 bis 300 mg in einer therapeutisch festgelegten Form zu verabreichen. Es besteht somit ein Bedarf an Antiulcusmitteln, die länger wirken und/ oder wirksamer sind als Cimetidin und Ranitidin.

Die erfindungsgemäßen Verbindungen zeigen aufgrund ihrer spezifischen $H_2$-antagonistischen Aktivität eine Hemmung der Magensäuresekretion, wenn diese durch Histaminagonisten stimuliert wird [Ash und Schild, "Brit. J. Pharmacol. Chemother.", 27, 427 (1966) und Black et al., "Nature", 236, 385 (1971)]. Die pharmakologische Aktivität dieser Verbindungen, wie sie genauer weiter unten beschrieben werden, kann nach einer modifizierten Methode gemäß der DE-OS 27 34 070 beim perfundierten Rattenmagen gezeigt werden oder durch die Ermittlung der $pA_2$-Werte in vitro am Meerschweinchenvorhof (vgl. Ariens, Molecular Pharmacology, Band 1, Academic Press, New York, 1964) nachgewiesen werden. Weiterhin kann die $H_2$-antagonistische Wirkung an wachen Heidenhain-Pouch-Hunden nach der Methode von Black et al., "Nature", 236, 385 (1971) gezeigt werden. Die neuen Verbindungen antagonisieren weiterhin die Histaminwirkung auf die Kontraktionsfrequenz des isolierten rechten Atriums des Meerschweinchens, aber sie beeinflussen nicht histamin-

induzierte Kontraktionen des isolierten, glatten gastrointestinalen Muskels, wenn diese durch $H_2$-Agonisten hervorgerufen werden.

Nachdem Hemmstoffe für Histamin-$H_2$-Rezeptoren sowohl bezüglich der basalen Magensäuresekretion als auch der durch Gastrin, Histamin, Methacholin oder Nahrung induzierten Magensäuresekretion Hemmwirkung besitzen, können sie für die Behandlung von peptischen Ulcera, die durch übermäßige Sekretion von Magensäure verursacht sind, und bei der Therapie hyperacider Gastritis verwendet werden.

Der Erfindung liegt die Aufgabe zugrunde, neue Hemmstoffe für Histamin-$H_2$-Rezeptoren mit verbesserter und/oder länger anhaltender Wirksamkeit zur Verfügung zu stellen.

Diese Aufgabe wird durch die Erfindung gelöst.

Gegenstand der Erfindung sind daher neue Sulfenamidderivate der allgemeinen Formel I

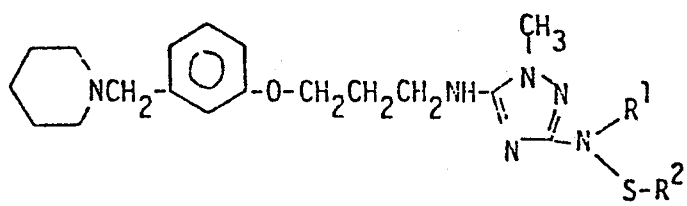

(I)

in der

$R^1$ für ein Wasserstoffatom

und

$R^2$ für lineares oder verzweigtkettiges $C_{1-6}$-Alkyl oder $C_{5-6}$-Cycloalkyl oder für unsubstituiertes oder ein- bis dreifach substituiertes Aryl oder Heteroaryl stehen

oder wobei

$R^1$ und $R^2$ zusammen einen Isothiazolinring oder Isothiazolinonring bilden,

sowie die physiologisch annehmbaren Salze davon.

In der allgemeinen Formel I steht $R^1$ für ein Wasserstoffatom. $R^2$ bedeutet lineares oder verzweigtkettiges $C_{1-6}$-Alkyl, wie zum Beispiel Methyl, Ethyl, Propyl, Isopropyl, Butyl, Isobutyl, n-Pentyl, Isopentyl, n-Hexyl und Isohexyl, oder $C_{5-6}$-Cycloalkyl, wie Cyclopentyl oder Cyclohexyl.

$R^2$ kann weiterhin für unsubstituiertes oder einfach bis dreifach substituiertes Aryl oder Heteroaryl stehen. Beispiele für Aryl sind: Phenyl, Naphthyl, Phenanthryl etc., wobei Phenyl bevorzugt wird. Der Arylrest kann unsubstituiert sein oder ein- bis dreifach substituiert sein, und zwar insbesondere durch Halogen, wie Chlor und Brom, Niedrigalkyl, insbesondere Methyl und Ethyl, Niedrigalkoxy, Trifluormethyl und Nitro.

Hierin werden unter "Niedrigalkyl" bzw. "Niedrigalkoxy" etc. Gruppen mit 1 bis 6 Kohlenstoffatomen im Alkylteil verstanden.

Eine bevorzugte Gruppe von erfindungsgemäßen Verbindungen ist dadurch gekennzeichnet, daß $R^1$ für ein Wasserstoffatom und $R^2$ für Methyl, Ethyl, Isopropyl oder Cyclohexyl stehen. Eine weitere bevorzugte Gruppe von erfindungsgemäßen Verbindungen ist dadurch gekennzeichnet, daß $R^1$ für ein Wasserstoffatom und $R^2$ für substituiertes oder mehrfach substituiertes Aryl stehen.

Hierbei kann beispielsweise der Phenylring durch lineares oder verzweigtkettiges Niedrigalkyl, vorzugsweise $C_{1-4}$-Alkyl, insbesondere durch eine Methylgruppe, substituiert sein. Die Alkylgruppe kann hierbei in ortho-, meta- oder para-Position, vorzugsweise in para-Position, gebunden sein. Ein weiterer bevorzugter Substituent ist die Trifluormethylgruppe, welche in ortho-, meta- oder para-Position des Phenylrings gebunden sein kann.

Weitere Substituenten am Phenylring sind Nitrogruppen und Halogenatome, insbesondere Chloratome, welche Substituenten unabhängig voneinander beispielsweise in ortho-, meta- oder para-Position gebunden sein können. Bei der Zweifachsubstitution sind die ortho-, para- (2,4-Stellung am Phenylring) bzw. die ortho-, ortho- (2,6-Stellung am Phenylring) Positionen bevorzugt. Bei der Dreifachsubstitution wird beispielsweise die 2,4,6-Position des Phenylrings bevorzugt.

$R^2$ kann weiterhin unsubstituiertes oder ein- bis dreifach substituiertes Heteroaryl bedeuten. Beispiele für Heteroarylgruppen sind: Pyridyl, Pyrimidinyl, Piperazinyl, Thiazolyl, Benzothiazolyl, Imidazolyl und Pyrazolyl, wobei Pyridyl, Pyrimidinyl und Benzothiazolyl bevorzugt werden. Die Heteroarylgruppen können unsubstituiert sein oder mit den gleichen Gruppen wie oben für Aryl beschrieben ein- bis dreifach substituiert sein. Insbesondere wird hierbei eine einfache Niedrigalkylsubstitution, insbesondere $C_{1-4}$-Substitution, in Betracht gezogen.

Schließlich können die Substituenten $R^1$ und $R^2$ miteinander einen Isothiazolinring oder Isothiazolinonring bilden.

Die erfindungsgemäßen Verbindungen werden durch ein Verfahren hergestellt, das dadurch gekennzeichnet ist, daß man ein Amin der Formel II

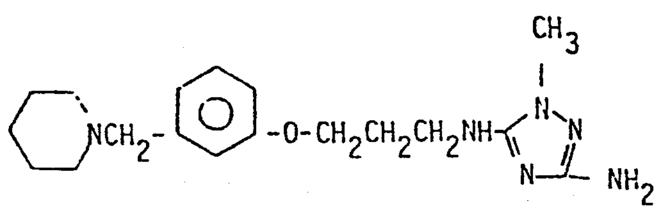

(II)

in an sich bekannter Weise in basenkatalytischer Reaktion

a) mit einem Sulfenylhalogenid der allgemeinen Formel III

$$R^2\text{-S-Hal} \qquad \text{(III)}$$

worin Hal für ein Chlor- oder für ein Bromatom steht und $R^2$ die oben angegebene Bedeutung hat, umsetzt und die erhaltene Verbindung gegebenenfalls in ihr physiologisch annehmbares Salz umwandelt

oder

b) mit einem Sulfenylsäurechlorid der Formel IV

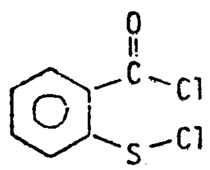

(IV)

umsetzt und die erhaltene Verbindung gegebenenfalls in ihr physiologisch annehmbares Salz umwandelt.

Die Amine der allgemeinen Formel II sind bekannte Verbindungen und werden zum Beispiel in der europäischen Offenlegungsschrift 0 057 564 beschrieben. Das gleiche gilt für die Sulfenylchloridderivate, die beispielsweise in E. Kühle, The Chemistry of Sulfenic Acids; Georg Thieme Verlag 1973 beschrieben werden.

Die Umsetzung erfolgt in einem inerten Lösungsmittel, wie Ether, Tetrahydrofuran oder Dioxan, vorzugsweise Tetrahydrofuran, bei einer Temperatur von 0°C bis 10°C, vorzugsweise bei 5°C. Als Base lassen sich tertiäre Amine, wie Dimethylbenzylamin, vorzugsweise Pyridin, verwenden. Die Aufarbeitung erfolgt in an sich bekannter Weise, beispielsweise durch Einengen des Reaktionsgemisches, Kristallisation und/oder Reinigung durch Säulenchromatographie.

Die Erfindung umfaßt auch die physiologisch annehmbaren Salze der genannten Verbindungen.

Diese Salze können zum Beispiel mit Mineralsäuren, wie Chlor-, Brom- und Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure etc., gebildet werden.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutisch annehmbaren Trägern oder Verdünnungsmitteln hergestellt werden.

Die erfindungsgemäßen Verbindungen können daher für die orale, bukkale, topische, parenterale oder rektale Verabreichung formuliert werden, wobei die orale Verabreichung bevorzugt wird. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die erfindungsgemäßen Verbindungen zeichnen sich gegenüber anerkannt guten Arzneimitteln der gleichen Wirkungsrichtung durch eine Verbesserung der pharmakologischen Aktivitäten aus. Dies ergibt sich aus den Ergebnissen der im folgenden dargestellten pharmakologischen Vergleichsuntersuchungen.

Eine anerkannte Methode zur Bestimmung $H_2$-antagonistischer Wirksamkeit ist die Ermittlung der $pA_2$-Werte in vitro am isolierten rechten Vorhof des Meerschweinchens.

| | $pA_2$-Werte | |
|---|---|---|
| Cimetidin: | 6.31 | Vergleich |
| Ranitidin: | 6.81 | Vergleich |
| Beispiel 1: | 8.80 | |

Andere Verbindungen der allgemeinen Formel I zeigen ähnliche pharmakologische Wirkungen.

## Beispiel 1

Herstellung von 1-Methyl-5-[3-[3-(1-piperidinylmethyl)phenoxy]-propylamino]-3-(4-methylbenzolsulfenamido)-1H-1,2,4-triazol

a) Herstellung von 2-[3-[3-(1-Piperidinylmethyl)phenoxy]propyl]-1H-isoindol-1,3-(2H)-dion

28.7 g (0.15 mol) 3-(1-Piperidinyl)phenol gelöst in 25 ml warmen abs. Ethanol werden zu einer Lösung aus 3.45 g (0.15 mol) Natrium in 25 ml abs. Ethanol getropft und 1 Stunde bei Raumtemperatur gerührt. Anschließend gibt man zur Reaktionslösung portionsweise 40.2 g (0.15 mol) N-(3-Brom-propyl)-phthalimid, erhitzt 6 Stunden auf Rückflußtemperatur und engt im Vakuum ein. Der Rückstand wird in Essigsäureethylester aufgenommen, mehrmals mit 1nNaOH-Lösung extrahiert, mit Wasser neutralgewaschen und über Natriumsulfat getrocknet. Das nach dem Einengen anfallende orange-gelbe Öl wird ohne Reinigung weiter umgesetzt.

Ausbeute: 45 g (79.3 % d.Th.)

b) Herstellung von 3-[3-(1-Piperidinylmethyl) phenoxy]propylamin

45 g (0.118 mol) 2-[3-[3-(1-Piperidinylmethyl) phenoxy]propyl]-1H-isoindol-1,3-(2H)-dion werden in 300 ml Ethanol gelöst, unter Rühren mit 16 ml (0.33 mol) einer 80 %-igen wäßrigen Lösung Hydrazinhydrat versetzt und 1 Stunde unter Rückfluß erhitzt. Der ausgefallene Feststoff wird abfiltriert, das Filtrat im Vakuum eingeengt, der Rückstand in Essigsäureethylester aufgenommen, mit Wasser neutralgewaschen und über Natriumsulfat getrocknet. Nach dem Einengen der organischen Phase wird der ölige Rückstand im Hochvakuum destilliert.

$Kp._{0,01} = 125 - 150\ ^{o}C$

Ausbeute: 19.4 g (66.3 % d. Th.)

c) **Herstellung von**

N-Cyano-N'-[3-[3-(1-piperidinylmethyl) phenoxy] propyl] -O-phenylisoharnstoff

8.22 g (33.1 mmol) 3-[3-(1-Piperidinylmethyl) phenoxy] propylamin in 70 ml Isopropanol werden bei Raumtemperatur unter Rühren mit 7.89 g (33.1 mmol) N-Cyano-diphenylimidocarbonat versetzt und 45 Minuten auf 50 °C erwärmt. Die Reaktionslösung wird im Vakuum eingeengt, der Rückstand in Essigsäureethylester gelöst und mit Ether ausgefällt.

Farblose Kristalle vom Schmelzpunkt 123 - 124 °C

Rf = 0.70 ($CH_2Cl_2$ / MeOH 70 : 30)

Ausbeute: 11.0 g (85 % d.Th.)

d) **Herstellung von**

1-Methyl-$N^5$-[3-[3-(1-piperidinylmethyl) phenoxy] propyl] -1H-1,2,4-triazol-3,5-diamin

3.92 g (10 mmol) N''-Cyano-N'-[3-(1-piperidinylmethyl) phenoxy] propyl -O-phenylisoharnstoff werden mit 0.57 g (13 mmol) Methylhydrazin 9 Stunden in 200 ml Isopropanol auf Rückflußtemperatur erhitzt. Nach dem Abkühlen der Reaktionslösung wird das Lösungsmittel im Vakuum entfernt und der Rückstand aus Ether gefällt.

Farblose Kristalle vom Schmelzpunkt 93 - 94 °C

Rf = 0.5 ($CH_3OH$ / conc. $NH_3$ 80 : 1)

Ausbeute: 1.79 g (52 % d.Th.)

e) Herstellung von

1-Methyl-5-[3-[3-(1-piperidinylmethyl)phenoxy]propylamino]-3-(4-methyl-benzolsulfenamido)-1H-1,2,4-triazol

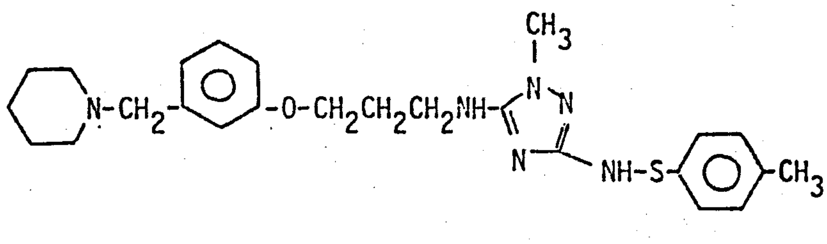

2.4 g (7.0 mmol) 1-Methyl-$N^5$-[3-[3-(1-piperidinylmethyl)phenoxy] propyl] -1H-1,2,4-triazol-3,5-diamin werden unter Feuchtigkeitsausschluß in 30 ml abs. Tetrahydrofuran gelöst, mit 0.85 ml (10.5 mmol) abs. Pyridin versetzt, auf $0^{o}$ C abgekühlt und tropfenweise mit einer Lösung aus 1.17 g (7.4 mmol) p-Tolylsulfenylchlorid in 5 ml abs. Tetrahydrofuran umgesetzt. Nach 1 Stunde Reaktionszeit bei Raumtemperatur wird die Reaktionslösung in 100 ml einer gesättigten wäßrigen $NaHCO_3$-Lösung gegossen und dreimal mit je 100 ml Essigsäureethylester extrahiert. Die organische Phase wird mit Wasser neutralgewaschen, über Natriumsulfat getrocknet und das Lösungsmittel im Vakuum eingeengt. Der Rückstand wird durch Säulenchromatographie ($Al_2O_3$ neutral; Elutionsmittel Essigsäureethylester) gereinigt.

Gelbliche Kristalle vom Schmelzpunkt 63 - 65 $^{o}$ C

Rf = 0.40 ($Al_2O_3$ neutral / Essigsäureethylester)

Ausbeute: 0.3 g (10 % d. Th.)

$C_{25}H_{34}N_6OS$ (467) Ber.: C 64.24 H 7.28 N 17.99 S 6.85

Gef.: C 63.73 H 7.17 N 17.53 S 6.73

$^1$H-NMR-Spektrum: ($CDCl_3$, TMS als interner Standard)

$\delta$ = 1.33 - 1.80 (m) ($-(CH_2)_3$) 6 H,
1.85 - 2.15 (m) ($-CH_2-$) 2 H,
2.27 (s) ($Ar-CH_3$) 3 H,
2.33 - 2.60 ($N(CH_2)_2-$) 4 H,
3.43 (s) ($-N-(CH_3)$ ) 3 H,
3.44 - 3.68 (m) ($-CH_2-$) ($N-CH_2$) 4 H,

4.03 (t) ($-OCH_2$) 2 H,
4.60 (t) (-NH) (austauschbar mit $D_2O$) 1 H,
6.67 - 7.40 (m) (Aromaten-H) (-N-H-S-)
(austauschbar mit $D_2O$)9 H ppm.

## Beispiel 2

1-Methyl-5-[3-[3-(1-piperidinylmethyl)phenoxy]propylamino]-3-(2-nitro-benzolsulfenamido)-1H-1,2,4-triazol

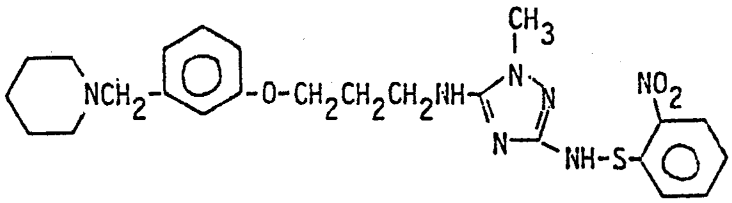

Die Herstellung erfolgt analog Beispiel 1 aus 2.4 g(7.0 mmol) 1-Methyl-$N^5$-[3-[3-(1-piperidinylmethyl)phenoxy]propyl]-1H-1,2,4-triazol-3,5-diamin und 1.40 g (7.4 mmol) 2-Nitro-benzolsulfenylchlorid.

Gelbliche Kristalle vom Schmelzpunkt 71 - 73 °C

Rf = 0.35 ($Al_2O_3$ neutral/Essigsäureethylester)

Ausbeute: 0.6 g (17.6 % d.Th.)

| | | | | |
|---|---|---|---|---|
| $C_{24}H_{31}N_7O_3S$ (498) | Ber.: | C 57.95 | H 6.28 | S 6.44 |
| | Gef.: | C 57.79 | H 6.49 | S 6.69 |

$^1$H-NMR-Spektrum: (d$_6$-DMSO, TMS als interner Standard)

δ = 1.23 - 1.63 (m) ($-(CH_2)_3-$) 6 H,
1.80 - 2.12 (m) ($-CH_2-$) 2 H,
2.20 - 2.43 (m) ($N(CH_2)_2$) 4 H,
3.30 (s) ($N-CH_3$) 3 H,
3.40 (s, breit) ($N-CH_2$) ($-CH_2-$) 4 H,
4.00 (t) ($-O-CH_2$) 2 H,
6.40 (t) (austauschbar mit $D_2O$) (-NH) 1 H,
6.70 - 7.90 (m) (Aromaten-H) 7 H
8.20 (s) (austauschbar mit $D_2O$) (NH-S) 1 H,
8.30 (d) (Aromaten-H) 1 H ppm.

## Beispiel 3

1-Methyl-5-[3-[3-(1-piperidinylmethyl) phenoxy]propylamino]-3-(2,4-dinitrobenzolsulfenamido)-1H-1,2,4-triazol

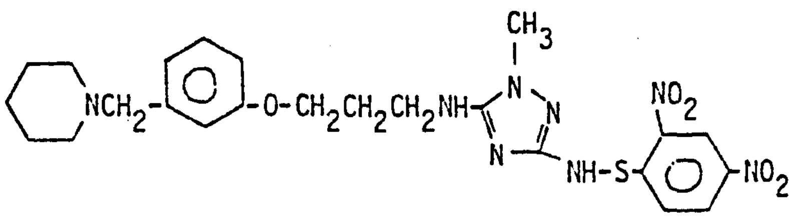

Die Herstellung erfolgt analog Beispiel 1 aus 2.4 g (7.0 mmol) 1-Methyl-$N^5$-[3-[3-(1-piperidinylmethyl) phenoxy]propyl]-1H-1,2,4-triazol-3,5-diamin und 1.73 g (7.4 mmol) 2,4-Dinitrobenzolsulfenylchlorid.

Gelbliche Kristalle vom Schmelzpunkt 149 - 150 °C

Rf = 0.25 ($Al_2O_3$ neutral/Essigsäureethylester)

Ausbeute: 2.55 g (67 % d.Th.)

$C_{24}H_{30}N_8O_5S$ (543)

| | C | H | N | S |
|---|---|---|---|---|
| Ber.: | 53.12 | 5.57 | 20,65 | 5,91 |
| Gef.: | 52.80 | 5.52 | 20,26 | 5,79 |

$^1$H-NMR-Spektrum: ($d_6$-DMSO, TMS als interner Standard)

$\delta$ = 1.23 - 1.67 (m) ( $(-CH_2-)_3$ ) 6 H,
1.83 - 2.13 (m) ($-CH_2-$) 2 H,
2.20 - 2.43 (m) ($-(CH_2)_2-$) 4 H,
3.13 - 3.50 (m) ($N-CH_2$) 2 H,
3.33 (s) ($N-CH_3$) 3 H,
3.43 (s) ($N-CH_2$) 2 H,
4.00 (t) ($O-CH_2$) 2 H,
6.47 (t) (N-H) (austauschbar mit $D_2O$) 1 H,
6.67 - 6.93 (m) (Aromaten-H) 3 H,
7.23 (t) (Aromaten-H) 1 H,
7.83 (d) (Aromaten-H) 1 H,

8.47 (s) (NH) (austauschbar mit $D_2O$)
8.57 (dd) (Aromaten-H) 1 H,
8.93 (d) (Aromaten-H) 1 H ppm.

Beispiel 4

1-Methyl-5-[3-[3-(1-piperidinylmethyl) phenoxy] propylamino]-3-(4-chlorbenzolsulfenamido)-1H-1,2,4-triazol

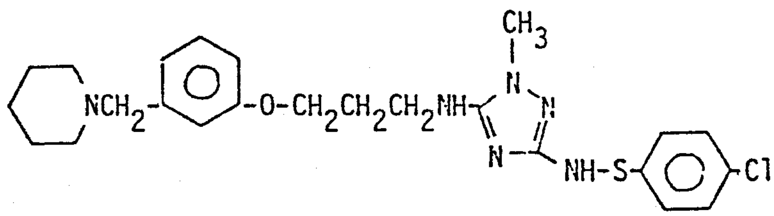

Die Herstellung erfolgt analog Beispiel 1 aus 2.4 g (7.0 mmol) 1-Methyl-$N^5$-[3-[3-(1-piperidinylmethyl) phenoxy] propyl] -1H-1,2,4-triazol-3,5-diamin und 1.32 g (7.4 mmol) 4-Chlorbenzolsulfenylchlorid

Gelbliche Kristalle vom Schmelzpunkt 47 - 48 °C

Rf = 0.3 ($Al_2O_3$ neutral/Essigsäureethylester)

Ausbeute: 0.6 g (20 % d.Th.)

$C_{24}H_{31}N_6OSCl$ (487)

$^1$H-NMR-Spektrum: (CDCl$_3$, TMS als interner Standard)

$\delta$ = 1.33 - 1.70 (m) ($-(CH_2-)_3$) 6 H,
1.83 - 2.10 (m) ($-CH_2-$) 2 H,
2.20 - 2.50 (m) ($-(CH_2)_2$) 4 H,
3.33 (s) ($N-CH_3$) 3 H,
3.33 - 3.53 (m) ($-CH_2-$) 2 H,
3.43 ($N-CH_2$) 2 H,

4.00 (t) (O-$C\underline{H}_2$) 2 H,

4.60 (t) (N-$\underline{H}$) (austauschbar mit $D_2O$) 1 H,

6.53 - 7.43 (m) (Aromaten-$\underline{H}$) 8 H,

8.00 (s) (N-$\underline{H}$) (austauschbar mit $D_2O$) 1 H ppm

Beispiel 5

1-Methyl-5-[3-[3-(1-Piperidinylmethyl)phenoxy]propylamino]-3-(3-trifluormethylbenzolsulfenamido)-1H - 1,2,4-triazol

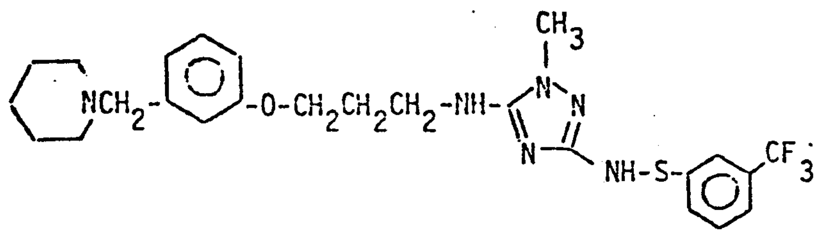

Die Herstellung erfolgt analog Beispiel 1 aus 2.4 g (7.0 mmol) 1-Methyl-$N^5$-[3[3-(1-piperidinylmethyl)phenoxy]-propyl]-1H - 1,2,4-triazol-3,5-diamin und 1.57 g (7.4 mmol) 3-Trifluormethyl-benzolsulfenylchlorid.

Amorpher Feststoff;

Rf = 0.48 ($Al_2O_3$ neutral/Essigsäureethylester)

Ausbeute: 0.4 g (10 % d. Th.)

$C_{25}H_{31}F_3N_6OS$ (521)

Ber.: C 57.68 H 6.00 N 16.14 S 6.16

Gef.: C 57.77 H 6.07 N 16.17 S 6.12

$^1$H-NMR-Spektrum: ($CDCl_3$, TMS als interner Standard)

$\delta$ = 1.20 - 1.67 (m) (-$(C\underline{H}_2)_3$) 6 H,

1.97 (m) (-$C\underline{H}_2$-) 2 H,

2.23 - 2.50 (m) (-$(C\underline{H}_2)_2$-) 4 H,

3.40 (s) (N-$C\underline{H}_3$) 3 H,

3.47 (s) (N-$C\underline{H}_2$) 2 H,

3.53 (t) (N-$C\underline{H}_2$) 2 H,

3.97 (t) (O-$C\underline{H}_2$) 2 H,

4.67 (t, breit) (N-H)

(austauschbar mit $D_2O$) 1 H,

6.57 - 7.63 (m) Aromaten-H) 8 H,

8.17 (s) (N-H) (austauschbar mit $D_2O$) 1 H ppm.

Beispiel 6

N-[[5-[3-[3-(1-Piperidinylmethyl) phenoxy]propylamino]-1-methyl-1H-1,2,4-triazol-3-yl]-1,2-benziso-thiazolin-3-on.

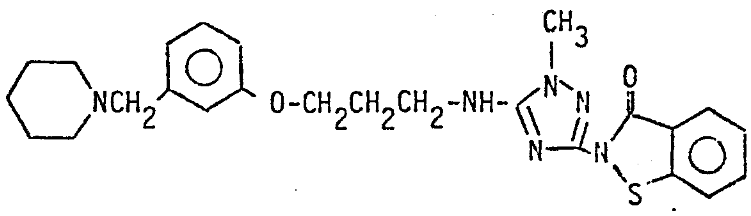

Die Herstellung erfolgt analog Beispiel 1 aus 2.4 g (7.0 mmol) 1-Methyl-$N^5$-[3[3-(1-piperidinyl-methyl) phenoxy] -propyl]-1H-1,2,4-triazol-3,5-diamin und 1.52 g (7.4 mmol) 2-Chlorcarbonylbenzolsulfenylchlorid.

Farblose Kristalle vom Schmelzpunkt 132 - 133°C.

Rf = 0.55 ($Al_2O_3$ neutral/Essigsäureethylester/Methanol 95:5)

Ausbeute: 0.39 g (15 % d. Th.)

| | | | | | |
|---|---|---|---|---|---|
| $C_{24}H_{30}N_6O_2S$ (467) | Ber.: | C 61.78 | H 6.48 | N 18.01 | S. 6.87 |
| | Gef.: | C 62.15 | H 6.22 | N 17.98 | S. 6.61 |

$^1$H-NMR-Spektrum: ($CDCl_3$, TMS als interner Standard)

$\delta$ = 1.33 - 1.70 (m) ($-(CH_2-)_3$) 6 H,
2.13 (m) ($-CH_2-$) 2 H,
2.23 - 2.47 (m) ($-(CH_2)_2-$) 4 H,
3.43 (s) ($N-CH_2-$) 2 H
3.60 (s) ($N-CH_3$) 3 H,
3.67 (t) ($N-CH_2$) 2 H,
4.13 (t) ($O-CH_2$) 2 H,
5.07 (t) (N-H) (austauschbar mit $D_2O$) 1 H,

6.67 - 8.12 (m) (Aromaten-H) 8 H ppm.

Beispiel 7

1-Methyl-5-[3-[3-(1-piperidinylmethyl)phenoxy]propylamino]-3-(2-pyridinsulfenamido)-1H-1,2,4-triazol

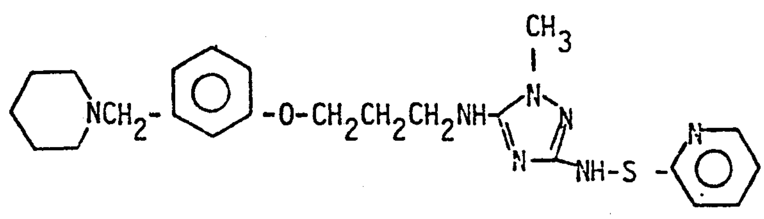

Die Herstellung erfolgt analog Beispiel 1 aus 2.4 g (7.0 mmol) 1-Methyl-$N^5$-[3-[3-(1-piperidinylmethyl) phenoxy]propyl]-1H-1,2,4-triazol-3,5-diamin und 1.07 g (7.4 mmol) 2-Pyridinsulfenylchlorid.

Farblose Kristalle vom Schmelzpunkt 141 - 142 °C

Rf = 0.2 ($Al_2O_3$ neutral / Essigsäureethylester)

Ausbeute: 0.67 g (21 % d.Th.)

$C_{23}H_{31}N_7OS$ (454) Ber.: C 60.90 H 6.85 N 21.62

Gef.: C 60.88 H 7.04 N 21.62

$^1$H-NMR-Spektrum: (CDCl$_3$, TMS als interner Standard)

$\delta$ = 1.33 - 1.73 (m) ($-(CH_2)_3-$) 6 H,
1.97 (m) ($-CH_2-$) 2 H,
2.27 - 2.50 (m) ($-(CH_2)_2-$) 4 H,

3.37 (s) ($N-C\underline{H}_3$) 3 H,

3.43 (s) ($-C\underline{H}_2-$) 2 H,

3.53 (t) ($N-C\underline{H}_2$) 2 H,

4.03 (t) ($O-C\underline{H}_2$) 2 H,

4.60 (t) (N-H) (austauschbar mit $D_2O$) 1 H,

6.67 - 7.63 (m) (Aromaten-H) 7 H,

7.67 (s) (N-H) (austauschbar mit $D_2O$) 1 H,

8.43 (d) (Aromaten-H) 1 H ppm.

Beispiel 8

3-(Cyclohexansulfenamido)-1-methyl-5-[3-[3-(1-piperidinylmethyl)phenoxy]propylamino]-1H-1,2,4-triazol

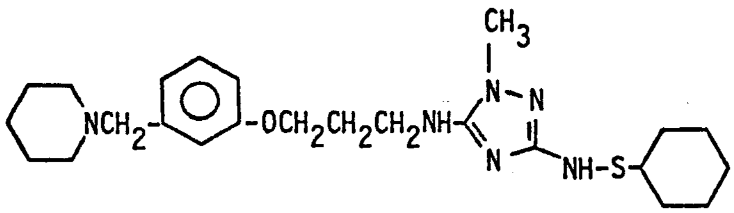

Die Herstellung erfolgt analog Beispiel 1 aus 2.4 g (7.0 mmol) 1-Methyl-$N^5$-[3-[3-(1-piperidinylmethyl)phenoxy]propyl]-1H-1,2,4-triazol-3,5-diamin und 1.11 g (7.4 mmol) Cyclohexansulfenylchlorid.

Öl

Rf = 0.25 ($Al_2O_3$ neutral/Essigsäureethylester)

Ausbeute: 0.29 g (9 % d.Th.)

$C_{24}H_{38}N_6OS$ (459)

| | | | | |
|---|---|---|---|---|
| Ber.: | C 62.85 | H 8.35 | N 18.32 | S 6.99 |
| Gef.: | C 62.98 | H 8.34 | N 17.74 | S 7.32 |

$^1$H-NMR-Spektrum: ($CDCl_3$, TMS als interner Standard)

$\delta$ = 1.17 - 2.17 (m) 18 H,
2.23 - 2.50 (m) ($N(C\underline{H}_2)_2$) 4 H,
2.97 (m) (-C$\underline{H}$) 1 H,
3.43 (s) ($N-C\underline{H}_2;N-CH_3$) 5 H,
3.60 (t) ($-C\underline{H}_2-$) 2 H,
4.13 (t) ($-O-C\underline{H}_2$)2 H,
4.33 (t) (austauschbar mit $D_2O$)(-N$\underline{H}$) 1 H,
5.33 (s) (austauschbar mit $D_2O$)(N$\underline{H}$-S) 1 H,
6.67 - 7.30 (m) (Aromaten-$\underline{H}$) 4 H ppm.

Beispiel 9

1-Methyl-5-[3-[3-(1-piperidinylmethyl)phenoxy]propylamino]-3-(methylsulfenamido)-1H-1,2,4-triazol

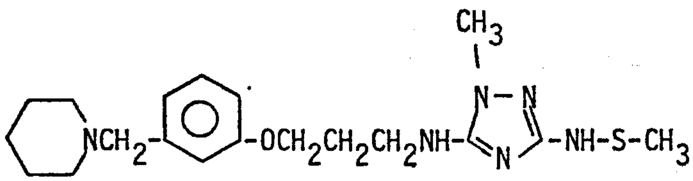

Die Herstellung erfolgt analog Beispiel 1 aus 2.4 g (7.0 mmol) 1-Methyl-$N^5$-[3-[3-(1-piperidinylmethyl)phenoxy]propyl]-1H-1,2,4-triazol-3,5-diamin und 0.6 g (7.4 mmol) Methylsulfenylchlorid.

Erstarrtes Öl

Rf = 0.27 ($Al_2O_3$ neutral / Essigsäureethylester)

Ausbeute: 0.3 g (11 % d.Th.)

$C_{19}H_{30}N_6OS$ (391)

$^1$H-NMR-Spektrum: (CDCl$_3$, TMS als interner Standard)

δ = 1.37 - 1.73 (m) (-($CH_2$)$_3$-) 6 H,
2.13 (m) (-$CH_2$-) 2 H,
2.40 (m) (N-($CH_2$)$_2$) 4 H,
2.43 (s) ($SCH_3$) 3 H,
3.45 (s, breit) (N-$CH_3$, N-$CH_2$) 5 H,
3.63 (t) (N-$CH_2$) 2 H,
4.13 (t) (O-$CH_2$) 2 H,
4.53 (t) (austauschbar mit $D_2O$) (N-H) 1 H,
5.93 (s) (austauschbar mit $D_2O$) (NH-S) 1 H,
6.67 - 7.33 (m) (Aromaten-H) 4 H ppm.

Beispiel 10

1-Methyl-5-[3-[3-(1-piperidinylmethyl)phenoxy]propylamino]-3-(2-chinolin-sulfenamido)-1H-1,2,4-triazol

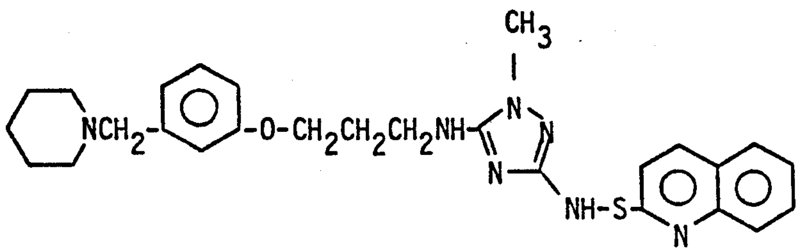

Die Herstellung erfolgt analog Beispiel 1 aus 2.4 g (7.0 mmol) 1-Methyl-$N^5$-[3-[3-(1-piperidinylmethyl)phenoxy]propyl]-1H-1,2,4-triazol-3,5-diamin und 1.44 g (7.4 mmol) 2-Chinolinsulfenylchlorid.

Amorpher Feststoff vom Schmelzpunkt 50 - 60 °C

Rf = 0.5 ($Al_2O_3$ neutral / Essigsäureethylester/Methanol 95:5)

Ausbeute: 0.28 g (8 % d.Th.)

$C_{27}H_{33}N_7OS$ (504)

$^1$H-NMR-Spektrum: (CDCl$_3$, TMS als interner Standard)

$\delta$ = 1.30 - 1.70 (m) ($-(CH_2)_3-$) 6 H,
1.93 (m) ($-CH_2-$) 2 H,
2.13 - 2.50 (m) ($N-(CH_2)_2-$) 4 H,
3.30 (s) ($N-CH_3$) 3 H,
3.43 (s) ($N-CH_2$) 2 H,
3.52 (t) ($N-CH_2$) 2 H,
3.93 (t) ($O-CH_2$) 2 H,
4.73 (t) (austauschbar mit $D_2O$) (N-H) 1 H,
6.55 - 8.13 (m) (Aromaten-H) 10 H,
8.24 (s) (austauschbar mit $D_2O$) (NH-S) 1 H ppm.

Beispiel 11

1-Methyl-5-[3-[3-(1-piperidinylmethyl)phenoxy]propylamino]-3-(2-pyrimidinsulfenamido)-1H-1,2,4-triazol

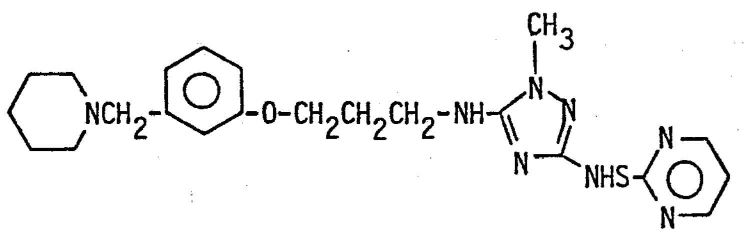

Die Herstellung erfolgt analog Beispiel 1 aus 2.4 g (7.0 mmol) 1-Methyl-$N^5$-[3-[3-(1-piperidinylmethyl)phenoxy]propyl]-1H-1,2,4-triazol-3,5-diamin und 0.98 g (7.4 mmol) 2-Pyrimidinsulfenylchlorid.

Farblose Kristalle vom Schmelzpunkt 119 - 121 °C

Rf = 0.43 (Kieselgel; $CH_2Cl_2$ / $NEt_3$ / $CH_3OH$ 90:10:1)

Ausbeute: 0.45 g (14 % d.Th.)

| | | | | | |
|---|---|---|---|---|---|
| $C_{22}H_{30}N_8OS$ (455) | Ber.: | C 58.13 | H 6.65 | N 24.65 | S 7.05 |
| | Gef.: | C 58.03 | H 6.67 | N 24.60 | S 6.94 |

$^1$H-NMR-Spektrum: ($d_6$-DMSO, TMS als interner Standard)

$\delta$ = 1.27 - 1.63 (m) ($-(C\underline{H}_2)_3-$) 6 H,
1.99 (m) ($-C\underline{H}_2-$) 2 H,
2.20 - 2.52 (m) ($N-(C\underline{H}_2)_2-$) 4 H,
3.30 (s) ($N-C\underline{H}_3$) 3 H,
3.37 (m) ($N-C\underline{H}_2$) 2 H,
3.45 (s) ($N-C\underline{H}_2$) 2 H,
4.03 (t) ($O-C\underline{H}_2$) 2 H,
6.37 (t) (austauschbar mit $D_2O$) (N-H) 1 H,
6.73 - 7.37 (m) (Aromaten-H) 5 H,
7.97 (s) (austauschbar mit $D_2O$) (NH-S) 1 H,
8.57 (d) (Aromaten-H) 2 H ppm.

Beispiel 12

1-Methyl-5-[3-[3-(1-piperidinylmethyl)phenoxy]propylamino]-3-(2-benzothiazolsulfenamido)-1H-1,2,4-triazol

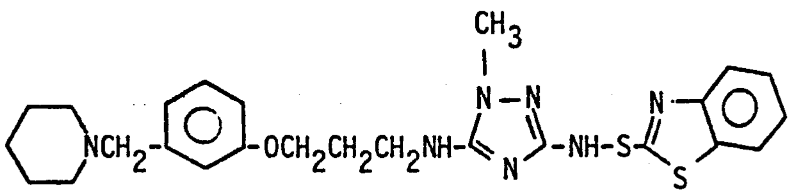

Die Herstellung erfolgt analog Beispiel 1 aus 2.4 g (7.0 mmol) 1-Methyl-$N^5$-[3-[3-(1-piperidinylmethyl)phenoxy]propyl]-1H-1,2,4-triazol-3,5-diamin und 1.49 g (7.4 mmol) 2-Benzothiazolsulfenylchlorid.

Farbloser amorpher Feststoff vom Schmelzpunkt 67 - 71 °C

Rf = 0.5 ($Al_2O_3$ neutral; Essigsäureethylester / Methanol 95:5)

Ausbeute: 0.54 g (15 % d.Th.)

| | | | | |
|---|---|---|---|---|
| $C_{25}H_{31}N_7OS_2$ (510) | Ber.: | C 58.91 | H 6.13 | N 19.24 |
| | Gef.: | C 58.70 | H 6.23 | N 19.06 |

$^1$H-NMR-Spektrum: ($CDCl_3$, TMS als interner Standard)

$\delta$ = 1.30 - 1.80 (m) ($-(C\underline{H}_2)_3-$) 6 H,
2.00 (m) ($-C\underline{H}_2-$) 2 H,
2.27 - 2.53 (m) ($N-(C\underline{H}_2)_2$) 4 H,
3.40 (s) ($N-C\underline{H}_3$) 3 H,
3.43 (s) ($N-C\underline{H}_2$) 2 H,
3.60 (t) ($N-C\underline{H}_2$) 2 H,
4.03 (t) ($OC\underline{H}_2$) 2 H,
4.67 (t) (austauschbar mit $D_2O$) (N-$\underline{H}$) 1 H,
6.60 - 7.97 (m) (Aromaten-$\underline{H}$) 8 H,
8.60 (s) (austauschbar mit $D_2O$) (N$\underline{H}$-S) 1 H ppm.

LUDWIG HEUMANN & CO. GMBH
8500 Nürnberg 1

Sulfenamidderivate, Verfahren zu ihrer Herstellung und diese Verbindungen enthaltende Arzneimittel

## PATENTANSPRÜCHE

1. Sulfenamidderivate der allgemeinen Formel I

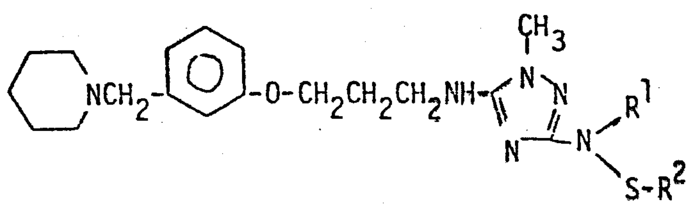

(I)

in der

$R^1$ für ein Wasserstoffatom
und
$R^2$ für lineares oder verzweigtkettiges $C_{1-6}$-Alkyl oder $C_{5-6}$-Cycloalkyl oder für unsubstituiertes oder ein- bis dreifach substituiertes Aryl oder Heteroaryl steht

oder wobei

$R^1$ und $R^2$ zusammen einen Isothiazolinring oder Isothiazolinonring bilden,
sowie die physiologisch annehmbaren Salze davon.

2. Verbindungen nach Anspruch 1, dadurch g e k e n n z e i c h n e t , daß $R^1$ für ein Wasserstoffatom und $R^2$ für lineares oder verzweigtkettiges $C_{1-6}$-Alkyl oder $C_{5-6}$-Cycloalkyl steht.

3. Verbindungen nach Anspruch 1, dadurch g e k e n n z e i c h n e t , daß $R^1$ für ein Wasserstoffatom und $R^2$ für unsubstituiertes oder ein- bis dreifach substituiertes Aryl steht.

4. Verbindungen nach Anspruch 1, dadurch g e k e n n z e i c h n e t , daß $R^1$ für ein Wasserstoffatom und $R^2$ für unsubstituiertes oder ein- bis dreifach substituiertes Heteroaryl steht.

5. Verbindungen nach Anspruch 1, dadurch g e k e n n z e i c h n e t , daß $R^1$ und $R^2$ miteinander einen Isothiazolinring oder Isothiazolinonring bilden.

6. 1-Methyl-5-[3-[3-(1-piperidinylmethyl)phenoxy]propylamino]-3-(4-methylbenzolsulfenamido)-1H-1,2,4-triazol und die physiologisch annehmbaren Salze davon.

7. 1-Methyl-5-[3-[3-(1-piperidinylmethyl)phenoxy]propylamino]-3-(4-chlorbenzolsulfenamido)-1H-1,2,4-triazol und die physiologisch annehmbaren Salze davon.

8. 1-Methyl-5-[3-[3-(1-piperidinylmethyl)phenoxy]propylamino]-3-(3-trifluormethylbenzolsulfenamido)-1H-1,2,4-triazol und die physiologisch annehmbaren Salze davon.

9. 1-Methyl-5-[3-[3-(1-piperidinylmethyl)phenoxy]propylamino]-3-(2-pyridinsulfenamido)-1H-1,2,4-triazol und die physiologisch annehmbaren Salze davon.

0141119

10. Verfahren zur Herstellung der Sulfenamidderivate nach den Ansprüchen 1 bis 9, dadurch gekennzeichnet, daß man ein Amin der Formel II

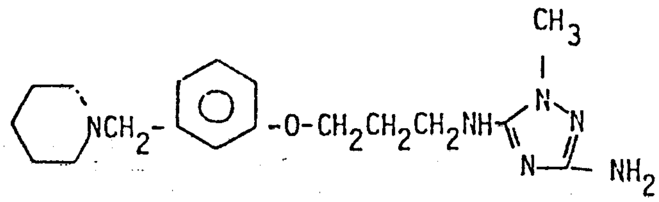

(II)

in an sich bekannter Weise in basenkatalysierter Reaktion

a) mit einem Sulfenylhalogenid der allgemeinen Formel III

$$R^2\text{-S-Hal} \quad \text{(III)}$$

worin Hal für ein Chlor- oder für ein Bromatom steht und $R^2$ die in Anspruch 1 angegebene Bedeutung hat, umsetzt und die erhaltene Verbindung gegebenenfalls in ihr physiologisch annehmbares Salz umwandelt

oder

b) mit einem Sulfenylsäurechlorid der Formel IV

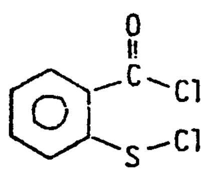

(IV)

umsetzt und die erhaltene Verbindung gegebenenfalls in ihr physiologisch annehmbares Salz umwandelt.

11. Arzneimittel, dadurch gekennzeichnet, daß es eine Verbindung nach den Ansprüchen 1 bis 9 und mindestens einen inerten, pharmazeutisch annehmbaren Träger oder ein inertes, pharmazeutisch annehmbares Verdünnungsmittel enthält.